# FASCICULE DE BREVET EUROPEEN

(11) **EP 3 612 471 B1**
(45) Date de publication et mention de la délivrance du brevet: **30.07.2025**
(21) Numéro de dépôt: 18711562.1
(22) Date de dépôt: 16.03.2018
(51) Int. Cl.: B65F 1/02, B65F 3/02, B65F 9/00, B65F 7/00

(54) **DISPOSITIF ET PROCÉDÉ DE STOCKAGE ET/OU DE TRANSPORT DE DÉCHETS ORGANIQUES**
VORRICHTUNG UND VERFAHREN ZUM LAGERN UND/ODER TRANSPORTIEREN VON ORGANISCHEM ABFALL
DEVICE AND METHOD FOR STORING AND/OR TRANSPORTING ORGANIC WASTE

(30) Priorité: 21.04.2017 LU 100173
(43) Date de publication de la demande: 26.02.2020
(73) Titulaire: Tank D S.A., 4370 Belvaux (LU)
(72) Inventeur: GOSSOT, Marie-Anne, 54500 Vandoeuvre-les-Nancy (FR)
(74) Mandataire: Ipsilon Benelux
(86) Numéro de dépôt international: PCT/EP2018/056713
(87) Numéro de publication internationale: WO 2018/192725

(56) Documents cités:
- EP-A1- 1 905 703
- WO-A1-2013/054871
- WO-A1-2017/072128
- CN-A- 104 843 401
- KR-A- 20110 112 530
- WOODARD, FRANK: "INDUSTRIAL WASTE TREATMENT HANDBOOK", REFEREX, 2001, USA, XP040425427, ISBN: 0-7506-7317-6
- ANONAS A V ET AL: "Evaluation of emission pattern, quantity and control of korean food waste composting odorants in a pilot-scale packed-bed scrubber", COMPENDEX, ENGINEERING INFORMATION, INC., NEW YORK, NY, US, 1 January 2007 (2007-01-01), XP002775991
- ANONAS A V ET AL: "Evaluation of emission pattern, quantity and control of korean food waste composting odorants in a pilot-scale packed-bed scrubber", 100TH ANNUAL CONFERENCE AND EXHIBITION OF THE AIR AND WASTE MANAGEMENT ASSOCIATION 2007, ACE 2007 2007 AIR AND WASTE MANAGEMENT ASSOCIATION USA, vol. 7, 2007, pages 4733 - 4746

## Description

### Domaine technique

L'invention a trait au domaine du stockage et du transport des déchets, plus particulièrement des déchets organiques, tels que les déchets ménagers ou alimentaires de la grande distribution et de restaurants ou de cantines de collectivités.

### Technique antérieure

Le document de brevet publié EP 1 905 703 A1 divulgue un véhicule, en l'occurrence un camion, spécialement équipé d'un dispositif de stockage et de transport de déchets organiques. Le camion comprend une cuve de stockage des déchets, ladite cuve étant généralement circulaire allongée disposée à l'horizontale. Le camion comprend également une goulotte de déversement équipée d'un broyeur et reliée au contenu de la cuve à déchets par une conduite. Les déchets organiques, du type déchets de cuisine, sont déposés dans la goulotte pour y être broyés par un broyeur et ensuite être aspirés vers la cuve par une dépression présente dans ladite cuve. Pour faire la dépression, une pompe à vide est reliée à l'enceinte de la cuve. Un réservoir d'eau est prévu entre le cabinet du camion et la cuve à déchets. Ce réservoir peut être chauffé. Cette eau est destinée au lavage de la cuve. Un réservoir auxiliaire pour récolter les huiles et graisses est prévu dans le réservoir à eau afin que ces huiles et graisses jouissent de la chaleur de l'eau et restent liquides et faciles à vidanger.

Le dispositif décrit ci-avant est intéressant en ce qu'il permet de collecter et de transporter des déchets organiques alimentaires. Le maintien de la cuve en dépression favorise le transfert de déchets depuis la goulotte de déversement. Cela permet également de limiter la vitesse de décomposition des déchets dans la cuve. Dans la pratique, un tel ralentissement n'est cependant pas particulièrement utile car le camion, s'il est utilisé de manière efficace à l'occasion d'une tournée de collecte, va se déplacer de place en place pour collecter les déchets sur un laps de temps réduit, typiquement un ou plusieurs jours, jusqu'à ce qu'il soit plein. Or ces déchets ne se décomposent pas en quelques jours, même par forte chaleur. Une fois la cuve remplie, le camion peut ensuite aller vider son contenu à un centre de traitement de déchets organiques. La dépression qui est réalisée dans la cuve semble servir essentiellement au transfert des déchets depuis la goulotte vers la cuve. Les gaz contenus dans la cuve et potentiellement malodorants sont aspirés par la pompe à vide et sont expulsées directement à l'atmosphère. Cela ne pose pas nécessairement de problème dans la mesure où le camion se déplace en principe de point de collecte en point de collecte. La solution de cet enseignement est intéressante dans une logique de collecte. Elle manque cependant d'être adéquate pour le stockage des déchets.

Le document de brevet publié WO 2014/059478 A1 divulgue un dispositif mobile de récupération et de traitement de déchets organiques du type alimentaire. Ce dispositif est monté sur un véhicule et comprend une goulotte de déversement des déchets, et un broyeur pourvu d'une alimentation en eau. L'eau chargée en déchets broyés est ensuite transférée vers un réservoir. Ce dispositif présente l'avantage de neutraliser les odeurs des déchets par leur traitement et incorporation dans de l'eau. Il présente également l'avantage que l'eau peut être utilisée, préalablement au mélange avec les déchets, pour nettoyer les conteneurs à déchets déversés dans la goulotte du dispositif. Il présente toutefois l'inconvénient qu'il requiert le stockage et le transport d'une grande quantité d'eau. De plus, les déchets sont sous forme de boue, ce qui n'est pas particulièrement adéquat pour leur valorisation notamment pour de la méthanisation et du compostage en fonction du taux de siccité.

Le document de brevet publié DE 203 17 332 U1 divulgue un dispositif de stockage et de transport de déchets organiques du type feuilles mortes. Le dispositif comprend, essentiellement, un conteneur fermé divisé en deux chambres, à savoir une chambre principale destinée au stockage des déchets et une chambre auxiliaire logeant un aspirateur dont la sortie est reliée à la chambre principale en vue d'y introduire les déches aspirés aux alentours du conteneur au moyen d'un tuyau flexible raccordé audit aspirateur.

Le document KR 2011 0112530 A décrit un autre exemple de dispositif de stockage de déchets organiques.

### Résumé de l'invention

### Problème technique

L'invention a pour objectif de pallier au moins un inconvénient de l'état de la technique, en l'occurrence de l'état de la technique susmentionné. Plus particulièrement, l'invention a pour objectif de favoriser la valorisation des déchets organiques, notamment du type ménager ou alimentaire.

### Solution technique

L'invention a pour objet un dispositif selon la revendication 1.

Selon un mode avantageux de l'invention, la ou chacune des colonnes de solution aqueuse présente une hauteur supérieure ou égale à 0.5 m, préférentiellement 1 m.

Selon un mode avantageux de l'invention, la ou chacune des colonnes de solution aqueuse présente une largeur ou un diamètre inférieur ou égal à 0.2 m, préférentiellement 0.1 m.

Selon un mode avantageux de l'invention, la ou les solutions aqueuses de la ou des colonnes, respectivement, comprennent une solution acide, préférentiellement d'un pH inférieur ou égal à 5 et/ou une solution basique, préférentiellement d'un pH supérieur à 9. La ou les solutions aqueuses peuvent comprendre du chlore, de l'eau oxygénée, et/ou de l'ozone.

Selon un mode avantageux de l'invention, l'unité de neutralisation des odeurs comprend plusieurs colonnes de solution aqueuse, lesdites colonnes étant reliées, d'un point de vue fluidique, en série de manière à ce que les gaz ayant circulé dans la solution aqueuse d'une des colonnes sortent par le haut de ladite colonne et soient introduits dans le bas d'une autre desdits colonnes de manière à circuler dans la solution aqueuse de ladite colonne.

Selon un mode avantageux de l'invention, les gaz circulent dans la ou les colonnes de solution aqueuse sous forme de bulles suivant une direction ascensionnelle sous l'effet de la poussée d'Archimède.

Selon un mode avantageux de l'invention, la ou chacune des colonnes de solution aqueuse comprend des moyens permettant de vidanger sa solution aqueuse, notamment dans la cuve à déchets. Ces moyens peuvent comprendre une ou plusieurs vannes fluidiquement raccordées à un point bas de chacune des colonnes et à la cuve à déchets.

Selon un mode avantageux de l'invention, le dispositif comprend un plateau supportant la cuve, la goulotte de déversement et l'unité de neutralisation des odeurs, ledit plateau étant configuré pour pouvoir être chargé sur un camion porte-berce équipé d'un bras mécanique hydraulique.

Selon un mode avantageux de l'invention, le plateau comprend à une extrémité un orifice apte à être engagé par un crochet du bras mécanique du camion porte-berce.

Selon un mode avantageux de l'invention, la conduite reliant la goulotte de déversement à la cuve à déchets débouche dans ladite cuve au travers d'une paroi inférieure de ladite cuve.

Selon un mode avantageux de l'invention, la conduite reliant la goulotte de déversement à la cuve à déchets débouche dans ladite cuve de manière sélective au travers d'une paroi inférieure de ladite cuve ou d'une paroi supérieure de ladite cuve.

Selon un mode avantageux de l'invention, la goulotte de déversement comprend un broyeur à déchets.

Selon un mode avantageux de l'invention, le dispositif comprend un système de levage et de basculement de conteneurs à déchets, apte à déverser de manière automatique lesdits déchets dans la goulotte de déversement.

Selon un mode avantageux de l'invention, le dispositif comprend une unité électrique de commande du dispositif configurée pour mettre en fonction la pompe à vide lorsque la dépression dans cuve à déchets est inférieure à 0.5 bar, préférentiellement 0.75 bar. La valeur de dépression est exprimée ici en valeur absolue, c'est-à-dire sans signe négatif.

Selon un mode avantageux de l'invention, la goulotte de déversement comprend un couvercle mobile entre une position ouverte et une position fermée, et le dispositif comprend une électrovanne située sur la conduite reliant la goulotte de déversement à la cuve à déchets, et des moyens électriques de commande de ladite électrovanne, lesdits moyens étant configurés pour ne permettre l'ouverture de ladite électrovanne que lorsque le couvercle est en position fermée.

Selon un autre mode avantageux de l'invention, la surpression tolérée avant l'ouverture de la vanne de surpression est comprise entre 0,08 et 0,12 bar.

Selon un mode avantageux de l'invention, le conduit d'amenée des produits est muni d'une vis sans fin ou d'une pompe péristaltique. La vis ou la pompe sont entrainées en rotation par un moteur. Le pas de la vis peut être variable avec un pas plus important en amont pour faciliter l'insertion des produits. Le conduit peut être agencé tel que l'amenée de produit se fait dans une partie basse de la cuve. Alternativement, complémentairement ou de façon sélective, l'amenée peut également se faire dans une partie haute de la cuve. Une vis flexible, c'est-à-dire dont l'axe n'est pas rectiligne peut être employée pour permettre le transport des produits dans un conduit non rectiligne.

Selon un mode avantageux de l'invention, des moyens de commande électrique et des moyens d'entrainement sont prévus pour commander la rotation de la vis ou de la pompe péristaltique. En particulier, la vis ou la pompe peuvent n'être actionnées en rotation que lorsque le couvercle de la goulotte de déversement est fermé.

Selon un mode avantageux de l'invention, la cuve est munie d'une vanne de vidange pour vider la cuve des produits qu'elle contient. Ainsi, les produits se présentant sous forme d'une soupe après fermentation peuvent être vidés de la cuve.

L'invention a également pour objet un procédé de stockage de déchets organiques, tels que des déchets alimentaires, comprenant les étapes suivantes : mise à disposition d'un dispositif de stockage des déchets ; déversement des déchets dans le dispositif de stockage ; remarquable en ce que le dispositif est conforme à l'invention.

Selon un mode avantageux de l'invention, le dispositif de stockage est maintenu stationnaire jusqu'à ce qu'il soit rempli, la durée de stationnement permettant aux déchets de se décomposer.

Selon un mode avantageux de l'invention, la durée de stationnement du dispositif est supérieure à 10 jours, préférentiellement 20 jours, plus préférentiellement plus de 30 jours.

Selon un mode avantageux de l'invention, le dispositif comprend une étape d'enlèvement du dispositif lorsqu'il atteint un niveau de remplissage prédéterminé, ledit enlèvement étant réalisé par chargement sur un camion porte-berce.

### Avantages de l'invention

Les mesures de l'invention sont intéressantes en ce qu'elles permettent d'éliminer de manière commode des déchets organiques, notamment pour des industries, restaurants, commerces ou cantines de collectivités, les déchets pouvant être éliminés au fur et à mesure de leur production, grâce au stationnement du dispositif à proximité du lieu de production des déchets. Le déversement des déchets et leur transfert par aspiration ou par la vis dans la cuve est particulièrement aisé. Les odeurs produites par la décomposition des déchets dans la cuve sont neutralisées de manière efficace. Le principe de lavage des gaz, notamment le lavage par oxydation et/ou acido-basique est particulièrement adapté à un fonctionnement sur une durée prolongée correspondant au stationnement du dispositif jusqu'à ce qu'il soit rempli, étant entendu que cette durée est variable et peut dans certains cas excéder plusieurs mois. Le couvercle de la goulotte de déversement et la logique de commande de l'aspiration des déchets ou de la vis, conditionnée par la fermeture du couvercle, procure un avantage important en termes de sécurité et de confort d'utilisation. Le fait de prévoir le dispositif sur un plateau du type berce est particulièrement intéressant pour le chargement et déchargement du dispositif. Avec le dispositif de l'invention, les déchets peuvent démarrer leur digestion anaérobie, les rendant plus intéressants pour leur revalorisation, notamment en vue de la production de méthane ou en vue de leur épandage comme compost.

### Brève description des dessins

La figure 1 est une vue en plan d'un dispositif de stockage et/ou de transport de déchets organiques selon un premier mode de réalisation de l'invention.
La figure 2 est une illustration schématique de l'unité de neutralisation des odeurs du dispositif de la figure 1.
La figure 3 est une illustration schématique du remplissage de la cuve du dispositif de la figure 1 et selon une configuration alternative.
La figure 4 est une vue en plan du dispositif de la figure 1 lors de son chargement ou déchargement sur un camion porte-berce.
La figure 5 est une vue en plan d'un dispositif de stockage et/ou de transport de déchets organiques selon un second mode de réalisation de l'invention

### Description d'un mode de réalisation

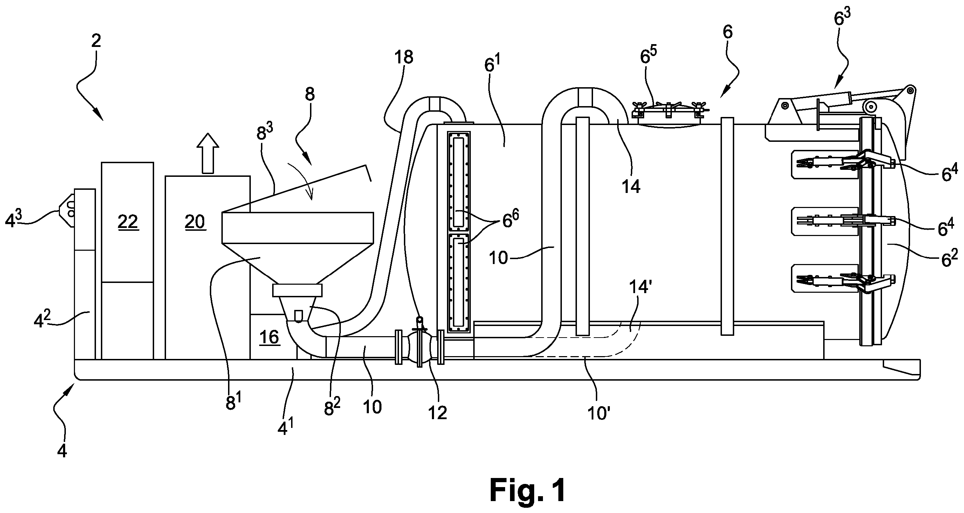

La figure 1 illustre un dispositif de stockage et/ou de transport de déchets organiques, tels que des déchets alimentaires. Le dispositif 2 comprend un plateau 4 avec une portion principale 4¹ généralement horizontale et destinée à recevoir les différents composants du dispositif, et une portion avant généralement verticale et équipée d'un moyen d'accrochage, tel qu'un anneau ou orifice 4³, avec un crochet d'un bras mécanique d'un camion berce en vue du déchargement et chargement du dispositif.

Le dispositif 2 comprend également une cuve 6 destinée à recevoir les déchets. La cuve 6 comprend une enveloppe principale 6¹, en l'occurrence généralement cylindrique et horizontale. Il est toutefois entendu que la cuve peut présenter d'autres formes. La cuve 6 comprend également un couvercle à son extrémité arrière. Ce dernier est monté pivotant par des moyens de pivotement et de commande du pivotement 6³. Il peut s'agit d'une charnière et d'un vérin hydraulique, bien connus en soi de l'homme de métier. Le couvercle 6² et l'enveloppe principale 6¹ de la cuve peuvent comprendre des moyens de fermeture et de mise en pression 6⁴ du couvercle contre l'enveloppe, afin de former une enceinte étanche. La cuve 6 peut également comprendre à son sommet une trappe d'accès 6⁵ pourvue également de moyens de fermeture et d'étanchéité. Une vanne (non représentée) peut également être prévue pour la mise à l'atmosphère de l'enceinte de la cuve. La cuve 6 peut également comprendre une ou plusieurs fenêtres 6⁶ disposées verticalement, destinées à permettre de visualiser le niveau de remplissage de la cuve.

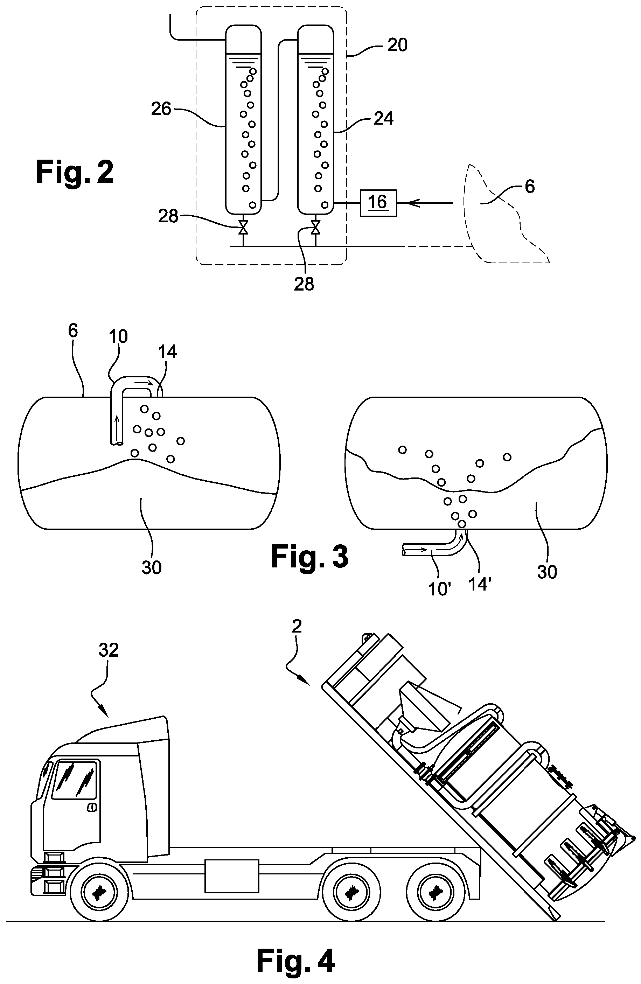

Le dispositif 2 comprend également une goulotte de déversement des déchets 8. Celle-ci comprend une goulotte 8¹ en tant que telle, optionnellement équipée en sa partie inférieure d'un broyeur 8². La goulotte 8¹ est surmontée d'un couvercle 8³ mobile, préférentiellement pivotant, entre une position ouverte et une position fermée. Elle peut prendre la forme d'une trappe ne permettant pas l'accès entre l'extérieur et l'intérieur de la goulotte, comme les trappes à container à vêtements. Ce type de trappe ou de sas permet de sécuriser l'accès à l'intérieur de la goulotte. La goulotte de déversement est reliée, d'un point de vue fluidique, à l'enceinte de la cuve à déchets 6 via la conduite 10. Une électrovanne 12 est disposée, d'un point de vue fluidique, le long de la conduite en question. La conduite 10 débouche à un point haut 14 de la paroi de la cuve 6. De manière alternative, éventuellement de manière sélective, ou encore de manière complémentaire, la conduite 10 peut également déboucher à un point bas 14' de la paroi de la cuve 6. L'intérêt de cette mesure sera détaillé en relation avec la figure 3.

Toujours en référence à la figure 1, le dispositif 2 comprend également une pompe à vide 16 raccordée, d'un point de vue fluidique, avec l'enceinte de la cuve 6 via une conduite 18. Un clapet anti-retour (non représenté) peut être prévu dans la conduite 18, notamment au niveau de la paroi de la cuve et/ou au niveau de la pompe 18.

La sortie de la pompe 16 est raccordée, d'un point de vue fluidique, avec une unité de neutralisation des odeurs 20. Les gaz contenus dans l'enceinte de la cuve peuvent contenir divers types de molécules comme notamment du sulfure d'hydrogène ou du mercaptan (composé organique comportant un groupement thiol -SH, c'est-à-dire un groupement sulfhydryle attaché à un atome de carbone) qui sont particulièrement malodorants. La sortie de la pompe à vide 16 est donc susceptible de produire un débit de gaz malodorant particulièrement gênant pour une utilisation stationnaire. Pour pallier ce problème, la sortie de la pompe à vide est raccordée, toujours d'un point de vue fluidique, avec l'unité de neutralisation des odeurs. Celle-ci peut fonctionner sur le principe d'adsorption, consistant à transférer le composé à éliminer de la phase gazeuse vers une phase solide. Le matériau le plus couramment utilisé est le charbon actif qui se présente sous différentes formes : grains, tissus... etc. Afin d'accroître les performances, le charbon est parfois imprégné d'aldéhyde notamment. Alternativement ou de manière complémentaire, on peut procéder à un lavage des gaz, c'est-à-dire qu'on procède au lavage du flux gazeux par une solution aqueuse par transfert des composés à éliminer de la phase gazeuse vers la phase liquide. Cette technique s'accompagne souvent d'une réaction chimique. Si le composé transféré ne subit aucune modification, seule l'absorption physique intervient. Si en phase liquide, pour améliorer la "solubilité apparente" du produit à éliminer, on joue sur le pH pour favoriser sa dissociation, le procédé est un transfert de masse accompagné d'une réaction chimique instantanée. L'unité de neutralisation des odeurs par lavage sera détaillée en relation avec la figure 2.

Le dispositif 2 peut comprendre un système de préhension, de levage et de basculement (non représenté) de conteneurs des déchets vers la goulotte 8¹ en tant que telle. Un tel système peut être animé par des vérins hydrauliques et/ou pneumatiques et/ou électriques. Il est destiné à faciliter la manipulation des conteneurs, en particulier la montée des déchets au-dessus de la goulotte 8¹ et leur basculement dans ladite goulotte.

Toujours en relation avec la figure 1, le dispositif 2 comprend une armoire électrique 22 avec des moyens de contrôle de la pompe à vide 16, du broyeur 8², de l'électrovanne 12 et éventuellement de l'unité de neutralisation des odeurs 20. Le dispositif 2 est alimenté en électricité via son armoire électrique 22 par un raccordement au réseau de distribution électrique. A cet effet, le dispositif, préférentiellement l'armoire électrique 22, comprend une prise électrique (non représentée) destinée à recevoir un câble d'alimentation électrique raccordé à une prise du réseau de distribution. Le dispositif 2 est en effet destiné à être disposé à proximité de bâtiments, notamment industriels ou de commerce, pourvus de prises d'alimentations électrique.

L'utilisation du dispositif 2 peut avoir lieu de la manière suivante : le couvercle 8³ de la goulotte de déversement 8 est soulevé par un opérateur afin de découvrir son ouverture. L'opérateur peut alors y déverser des déchets organiques, notamment du type alimentaire, par exemple au moyen d'un conteneur du type seau. Une fois les déchets déversés dans la goulotte 8, le couvercle 8³ est amené en position de fermeture de la goulotte 8. Alternativement, un conteneur, notamment à roulettes, peut être engagé à un système de levage et de basculement en vue du déversement automatique des déchets dans la goulotte 8¹. Un opérateur peut alors actionner le mouvement ascendant du système à l'aide d'une manette à action maintenue. Le couvercle de la goulotte étant couplé à l'action du système de basculement s'ouvre afin de permettre le déversement du contenu du conteneur. Par action maintenue inverse de la commande du système de basculement, le conteneur redescend, provoquant la fermeture du couvercle de la goulotte

L'opérateur peut alors actionner un bouton ou un moyen de commande quelconque (non représenté) afin d'activer le broyage des déchets au moyen du broyeur 8². Le broyage peut également être activé automatiquement par une détection de déchets. Le broyage est toutefois optionnel, si bien que l'opération de broyage décrite ci-avant est également optionnelle. L'électrovanne 12 est ensuite actionnée pour mettre la goulotte en communication fluidique avec l'enceinte en dépression de la cuve 6. S'en suit alors une aspiration des déchets via la conduite 10 vers l'intérieur de la cuve 6. L'ouverture de l'électrovanne 12 peut être sujette à une limitation de temps d'ouverture afin d'éviter de mettre l'enceinte de la cuve à la pression atmosphérique. Cette durée peut être comprise entre 2 et 60 secondes.

Un capteur de pression (non représenté) est prévu dans l'enceinte de la cuve. Le signal de pression de ce capteur est transmis à l'armoire électrique 22 qui va alors commander la mise en marche et l'arrêt de la pompe à vide 16 de manière à maintenir une dépression (par rapport à la pression atmosphérique) comprises entre 0.5 et 0.9 bar. Une temporisation, par exemple de quelques secondes, peut être prévue lorsque la dépression diminue au point d'atteindre la valeur minimale. En référence à la plage susmentionnée, la valeur minimale est de 0.5 bar.

La figure 2 illustre une unité de neutralisation des odeurs 20 du dispositif de la figure 1, fonctionnant sur le principe de lavage des gaz, plus précisément sur le principe de lavage acido-basique.

L'unité de neutralisation des odeurs 20 comprend une première colonne à bulles 24 de solution aqueuse, en l'occurrence de solution acide dont le pH peut être inférieur à 5, préférentiellement à 4. Les gaz en sortie de la pompe à vide 16 sont introduits en bas de la première colonne 24 afin de former des bulles dans la solution aqueuse acide et remonter vers la surface. Durant ce trajet, le gaz est en contact intime avec la solution et certaines molécules, notamment les mercaptans réagissent avec la solution et réalisent un transfert de masse vers la solution. Le flux gazeux produit à la surface de la solution aqueuse de la première colonne 24 est guidé par une conduite vers le bas d'une deuxième colonne à bulles 26 à solution aqueuse, celle-ci étant cette fois basique, par exemple avec un pH supérieur à 8, préférentiellement 9. Similairement à la première colonne, les gaz vont former des bulles dans la solution aqueuse basique et seront en contact intime avec celle-ci durant leur parcours vers la surface. Des réactions chimiques peuvent avoir lieu et réaliser des transferts de masse. Le gaz quittant la surface de la solution aqueuse de la deuxième colonne 26 peut alors être évacué à l'atmosphère.

Dans le cadre de la neutralisation des odeurs, une étape d'oxydation peut être superposée ou suivre le lavage acido-basique décrit ci-avant. L'opération est alors soit un lavage oxydant, soit un lavage acido-basique suivi d'une oxydation. Le fait d'oxyder le produit absorbé permet de régénérer en continu la solution de lavage.

Les solutions aqueuses de lavage peuvent comprendre du chlore, de l'eau oxygénée, et/ou de l'ozone.

A la figure 2, seules deux colonnes sont représentées. Il est entendu que davantage de colonnes peuvent être prévues, reliées entre-elles en série ou en parallèle.

Les colonnes 24 et 26 peuvent comprendre chacune un robinet ou une vanne 28 de vidange de la solution aqueuse. La sortie de cette ou ces vannes peut être reliée à une évacuation ou à la cuve 6. En effet, ces solutions aqueuses peuvent être ajoutées aux déchets sans pour autant perturber leur processus de dégradation et de fermentation.

Les colonnes 24 et 26 peuvent présenter une hauteur supérieure ou égale à 0.5 m, préférentiellement 1m et présenter une largeur ou un diamètre inférieur ou égal à 0.2 m, préférentiellement 0.1 m.

La figure 3 illustre deux configurations de remplissage de la cuve 3 du dispositif 2 de la figure 1.

A la configuration de gauche, les déchets sont acheminés par le point haut de la cuve 6, formant alors un amas 30 pouvant former un sommet en face de l'orifice 14 où débouche la conduite 10. Durant la fermentation des déchets, une phase liquide peut se former et l'amas peut alors s'aplanir, permettant alors un remplissage satisfaisant.

A la configuration de droite, les déchets sont acheminés par le point bas de la cuve 6. En fonction de la vitesse d'arrivée de déchets et de l'épaisseur de l'amas 30 en face de l'orifice 14', les déchets peuvent être projetés, notamment sur les côtés et favoriser le remplissage des côtés de la cuve 6. Il peut donc être intéressant de sélectionner une entrée des déchets dans la cuve par le haut et/ou par le bas, en fonction du profil de remplissage et de la nature des déchets. Dans le cadre de déchets à tendance sèche, il peut être avantageux d'utiliser l'entrée des déchets par le bas alors que pour les déchets à tendance humide, l'entrée par le haut peut être avantageuse.

La figure 4 illustre un camion 32 dans une configuration de chargement ou de déchargement du dispositif 2 de la figure 1, illustrant la facilité de manutention du dispositif, similairement aux compacteurs de cartons.

Le dispositif qui vient d'être décrit est particulièrement adapté pour stationner durant plusieurs semaines, voire plusieurs mois, auprès d'une entreprise, un commerce ou encore une cantine de collectivité (école, hôpital, etc.) produisant une certaine quantité de déchets organiques, notamment du type alimentaire. Ces déchets peuvent alors être régulièrement déversés dans la goulotte et ensuite aspirés sans difficulté et sans production d'odeurs nauséabondes, de coulée ou de développement de nuisibles (insectes, rats, ...). De plus, le fait que la cuve soit en permanence en dépression assure une fermentation contrôlée des déchets, les préparant de manière particulièrement avantageuse pour leur revalorisation, notamment par méthanisation.

Pour ces industries, commerces ou collectivités, il suffit de commander la livraison d'un tel dispositif, notamment sous forme de location. Son remplacement par un autre, vide, ne sera nécessaire que lorsque le premier sera plein. La dépression dans la cuve permet de conserver et de faire fermenter les déchets sur plusieurs mois, en l'absence de réfrigération des déchets, et même dans des conditions estivales particulièrement chaudes. Les trajets en camion sont par conséquent réduits et les déchets sont alors naturellement préparés pour leur valorisation.

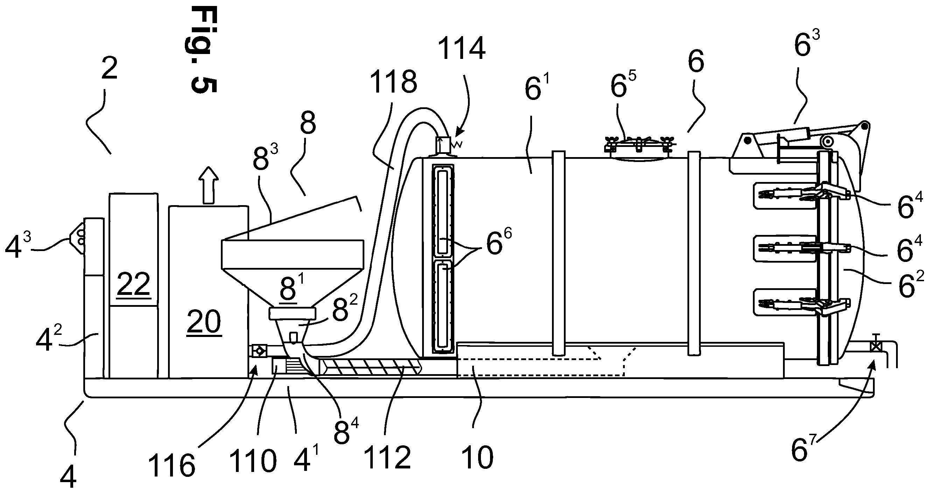

La figure 5 représente un second mode de réalisation d'un dispositif conforme à l'invention. Dans ce mode de réalisation, les gaz formés dans cuve ne sont pas aspirés par une pompe. La cuve n'est pas en dépression. Au contraire, la cuve est en légère surpression. Le dispositif de la figure 5 est dans l'ensemble identique à celui de la figure 1. Nous détaillons ici uniquement les différences entre ces deux dispositifs.

Le dispositif comprend une vanne de surpression 114. Celle-ci est normalement fermée. Elle s'ouvre sous l'action de la pression des gaz, lorsque la pression dépasse une certaine valeur seuil dans la cuve, de l'ordre de 0.08 à 0.12 bar au-dessus de la pression atmosphérique, par exemple 0.1 bar.

Les gaz parcourent le conduit 118 jusqu'à l'unité de neutralisation des odeurs 20. Un clapet anti-retour 116 est prévu pour empêcher le gaz de retourner dans la cuve. L'unité de neutralisation des odeurs 20 est conforme à ce qui est décrit plus haut, en particulier en lien avec la figure 2, mais sans la pompe d'aspiration. Dans le dispositif en surpression de la figure 5, le débit des gaz est plus faible qu'avec l'utilisation d'une pompe d'aspiration. Il en résulte un lavage des gaz ou une neutralisation des odeurs plus efficace.

Le dispositif de la figure 5 comprend une goulotte 8 munie optionnellement d'un broyeur 8². La goulotte peut comprendre une trémie pour déverser une grande quantité de produits. Similairement au mode de réalisation de la figure 1, la goulotte comporte un couvercle 8³. Alternativement, la goulotte peut être prévue sous la forme d'une trappe pivotante formant un sas qui empêche l'utilisateur d'être directement au contact avec le broyeur 8².

Sous le broyeur 8² se trouve une vanne guillotine 8⁴ qui s'ouvre à la mise en route du broyeur et qui permet d'assurer l'étanchéité de la cuve lorsque le couvercle 8³ est ouvert et lorsque le broyeur 8² est à l'arrêt.

Ensuite, les déchets introduits dans la goulotte de déversement 8 sont acheminés jusqu'à la cuve par une vis sans fin 112 qui est installée dans le conduit d'amenée 10. La vis sans fin 112 est entrainée par un moteur 110. Le moteur 110 régule la vitesse de rotation de la vis 112. Pour se prémunir d'un bouchon dans le conduit 10 qui serait dû à des produits coincés, il peut être prévu un mode de fonctionnement dégradé, dans lequel la vis 112 peut tourner dans un sens contraire au sens normal de fonctionnement. Aussi, la vis 112 peut avoir un pas de vis qui n'est pas constant sur toute sa longueur. Par exemple, un pas de vis plus grand à proximité de la goulotte permet d'éviter la création d'un bouchon à cet endroit. Lorsqu'il est choisi, pour des raisons d'encombrement ou autre, que le conduit 10 ne soit pas rectiligne, la vis peut être flexible. Ainsi, elle peut être formée intégralement d'un matériau déformable pour permettre le convoyage des produits le long du conduit non rectiligne. La vis 112 peut alimenter la cuve dans sa partie basse comme représenté sur la figure 5, ou dans sa partie haute (non représenté).

Le dispositif contient des moyens de commande 22 qui permettent de contrôler le fonctionnement de la vis 112 et du broyeur 8² en fonction de l'ouverture ou la fermeture du couvercle 8³, ou sous l'actionnement d'un moyen de commande de type bouton ou autre par l'utilisateur.

Alternativement à la vis sans fin 112, une pompe péristaltique peut entraîner les déchets jusqu'à la cuve.

La cuve 6 comporte une vanne de vidange 6⁷ pour vider la cuve des produits fermentés, devenus liquides. Celle-ci peut par exemple être actionnée à distance depuis la cabine du camion. Une trappe d'accès 6⁵ est prévue sur le haut de la cuve. Celle-ci peut aussi être agencée en bas à l'arrière de la cuve ou tout autre endroit approprié.

Le mode de fonctionnement en surpression du dispositif de la figure 5 est particulièrement avantageux pour le traitement des odeurs. Il est aussi de conception simple, moins encombrant et peu gourmand en énergie.

## Revendications

1. Dispositif (2) de stockage et/ou de transport de déchets organiques, tels que des déchets alimentaires, le dispositif comprenant :
- une cuve hermétique (6) destinée à recevoir les déchets ;
- une goulotte de déversement (8) des déchets, reliée d'un point de vue fluidique par une conduite (10, 10') à la cuve à déchets ;
- des moyens d'évacuation (16, 18, 114, 116, 118) des gaz présents dans ladite cuve comprenant, en outre,
une unité de neutralisation d'odeurs (20) émises par lesdits gaz, l'unité de neutralisation d'odeurs (20) étant reliée d'un point de vue fluidique à la cuve (6) par lesdits moyens d'évacuation (16, 18, 114, 116, 118) et comprenant au moins une colonne de solution aqueuse (24, 26), l'unité de neutralisation d'odeurs (20) étant configurée pour recevoir les gaz de la cuve (6) dans le bas de ladite ou desdites colonnes (24, 26),
les moyens d'évacuation des gaz comprenant, dans un premier mode de réalisation:
- une pompe à vide (16) reliée, d'un point de vue fluidique (18), à la cuve à déchets (6) et configurée pour aspirer les gaz présents dans ladite cuve et créer une dépression dans ladite cuve ; ou, dans un deuxième mode de réalisation:
- une vanne de surpression (114) configurée pour ne laisser passer les gaz de la cuve (6) vers l'unité de neutralisation des odeurs (20) que lorsque la pression des gaz dans la cuve est supérieure à une valeur seuil ;
les moyens d'évacuation des gaz comprenant, dans le deuxième mode de réalisation, en outre un clapet anti-retour (116) ne permettant la circulation des gaz que dans le sens de la cuve (6) vers l'unité de neutralisation des odeurs (20).

2. Dispositif (2) selon la revendication 1, **caractérisé en ce que** la ou chacune des colonnes de solution aqueuse (24, 26) présente une hauteur supérieure ou égale à 0.5 m, préférentiellement 1 m, et/ou la ou chacune des colonnes de solution aqueuse (24, 26) présente une largeur ou un diamètre inférieur ou égal à 0.2 m, préférentiellement 0.1 m.

3. Dispositif (2) selon l'une des revendications 1 ou 2, **caractérisé en ce que** la ou les solutions aqueuses de la ou des colonnes, respectivement, comprennent une solution acide, préférentiellement d'un pH inférieur ou égal à 5 et/ou une solution basique, préférentiellement d'un pH supérieur à 9.

4. Dispositif (2) selon l'une des revendications 1 à 3, **caractérisé en ce que** l'unité de neutralisation des odeurs (20) comprend plusieurs colonnes de solution aqueuse (24, 26), lesdites colonnes étant reliées en série de manière à ce que les gaz ayant circulé dans la solution aqueuse d'une (24) des colonnes sortent par le haut de ladite colonne (24) et soient introduits dans le bas d'une autre (26) desdits colonnes de manière à circuler dans la solution aqueuse de ladite colonne (26) ; et/ou **caractérisé en ce que** les gaz circulent dans la ou les colonnes de solution aqueuse (24, 26) sous forme de bulles suivant une direction ascensionnelle sous l'effet de la poussée d'Archimède ; et/ou **caractérisé en ce que** la ou chacune des colonnes de solution aqueuse (24, 26) comprend des moyens permettant de vidanger sa solution aqueuse, notamment dans la cuve à déchets.

5. Dispositif (2) selon l'une des revendications 1 à 4, **caractérisé en ce qu'**il comprend un plateau (4) supportant la cuve (6), les moyens d'évacuation des gaz (16, 18, 114, 116, 118), la goulotte de déversement (8) et l'unité de neutralisation des odeurs (20), ledit plateau (4) étant configuré pour pouvoir être chargé sur un camion porte-berce (32) équipé d'un bras mécanique hydraulique, le plateau (4) comprenant optionnellement, à une extrémité un orifice (4³) apte à être engagé par un crochet du bras mécanique du camion porte-berce (32).

6. Dispositif (2) selon l'une des revendications 1 à 5, **caractérisé en ce que** la conduite (10, 10') reliant la goulotte de déversement (8) à la cuve à déchets (6) débouche dans ladite cuve (6) au travers (14') d'une paroi inférieure de ladite cuve.

7. Dispositif (2) selon l'une des revendications 1 à 5, **caractérisé en ce que** la conduite (10, 10') reliant la goulotte de déversement (8) à la cuve à déchets (6) débouche dans ladite cuve (6) de manière sélective au travers (14') d'une paroi inférieure de ladite cuve ou au travers (14) d'une paroi supérieure de ladite cuve.

8. Dispositif (2) selon l'une des revendications 1 à 7, **caractérisé en ce que** la goulotte de déversement (8) comprend un broyeur à déchets (8²) ; et/ou **caractérisé en ce que** le dispositif (2) comprend un système de levage et de basculement de conteneurs à déchets, apte à déverser de manière automatique lesdits déchets dans la goulotte de déversement (8).

9. Dispositif (2) selon l'une des revendications 1 à 8, **caractérisé en ce qu'**il comprend une unité électrique (22) de commande du dispositif configuré pour mettre en fonction la pompe à vide (16) lorsque la dépression dans cuve à déchets (6) est inférieure à 0.5 bar, préférentiellement 0.75 bar.

10. Dispositif (2) selon l'une des revendications 1 à 9, **caractérisé en ce que** la goulotte de déversement (8) comprend un couvercle (8³) mobile entre une position ouverte et une position fermée, et le dispositif (2) comprend une électrovanne (12) située sur la conduite (10, 10') reliant la goulotte de déversement (8) à la cuve à déchets (6), et des moyens électrique (22) de commande de la dite électrovanne (12), lesdits moyens (22) étant configurés pour ne permettre l'ouverture de ladite électrovanne (12) que lorsque le couvercle (8³) est en position fermée.

11. Dispositif (2) selon l'une des revendications 1 à 10, **caractérisé en ce que** la valeur seuil est comprise entre 0,08 et 0,12 bar au-dessus de la pression atmosphérique.

12. Dispositif (2) selon l'une des revendications 1 à 11, **caractérisé en ce que** la conduite (10) comprend une vis sans fin (112) ou une pompe péristaltique et optionnellement **caractérisé en ce que** la goulotte de déversement (8) comprend un couvercle (8³) mobile entre une position ouverte et une position fermée, et le dispositif comprend des moyens électriques de commande (22) et d'entrainement (110) de ladite vis (112) ou de ladite pompe péristaltique, lesdits moyens (22, 110) étant configurés pour n'entrainer la vis ou la pompe péristaltique en rotation que lorsque le couvercle (8³) est en position fermée.

13. Procédé de stockage de déchets organiques, tels que des déchets alimentaires, comprenant les étapes suivantes :
- mise à disposition d'un dispositif de stockage des déchets ;
- déversement des déchets dans le dispositif de stockage ;
**caractérisé en ce que** le dispositif (2) est conforme à l'une des revendications 1 à 12.

14. Procédé selon la revendication 13, **caractérisé en ce que** le dispositif de stockage (2) est maintenu stationnaire jusqu'à ce qu'il soit rempli, la durée de stationnement permettant aux déchets de se décomposer, la durée de stationnement du dispositif (2) étant optionnellement supérieure à 10 jours, préférentiellement 20 jours, plus préférentiellement plus de 30 jours.

15. Procédé selon l'une des revendications 13 à 14, **caractérisé en ce qu'**il comprend une étape d'enlèvement du dispositif (2) lorsqu'il atteint un niveau de remplissage prédéterminé, ledit enlèvement étant réalisé par chargement sur un camion porte-berce (32).

## Patentansprüche

1. Vorrichtung (2) zur Lagerung und/oder zum Transport von organischen Abfällen, wie Lebensmittelabfällen, wobei die Vorrichtung umfasst:
- einen hermetischen Behälter (6) zur Aufnahme der Abfälle;
- einen Einfülltrichter (8) für die Abfälle, der fluidisch durch eine Leitung (10, 10') mit dem Abfallbehälter verbunden ist;
- Entlüftungsmittel (16, 18, 114, 116, 118) für die in dem Behälter vorhandenen Gase,
wobei die Vorrichtung ferner umfasst:
eine Einheit zur Geruchsneutralisation (20) der von den Gasen abgegebenen Gerüche, wobei die Einheit zur Geruchsneutralisation (20) fluidisch durch die Entlüftungsmittel (16, 18, 114, 116, 118) mit dem Behälter (6) verbunden ist und mindestens eine Säule mit wässriger Lösung (24, 26) umfasst, wobei die Einheit zur Geruchsneutralisation (20) so konfiguriert ist, dass sie die Gase aus dem Behälter (6) in den unteren Bereich der Säule(n) (24, 26) aufnimmt,
wobei die Entlüftungsmittel in einer ersten Ausführungsform umfassen:
- eine Vakuumpumpe (16), die fluidisch (18) mit dem Abfallbehälter (6) verbunden ist und so konfiguriert ist, dass sie die in dem Behälter vorhandenen Gase absaugt und im Behälter einen Unterdruck erzeugt; oder in einer zweiten Ausführungsform:
- ein Überdruckventil (114), das so konfiguriert ist, dass es die Gase aus dem Behälter (6) nur dann zur Einheit zur Geruchsneutralisation (20) durchlässt, wenn der Gasdruck im Behälter einen Schwellenwert überschreitet;
wobei die Entlüftungsmittel in der zweiten Ausführungsform ferner ein Rückschlagventil (116) umfassen, das den Gasfluss nur in Richtung vom Behälter (6) zur Einheit zur Geruchsneutralisation (20) ermöglicht.

2. Vorrichtung (2) nach Anspruch 1, **dadurch gekennzeichnet, dass** die oder jede Säule mit wässriger Lösung (24, 26) eine Höhe von mindestens 0,5 m, vorzugsweise 1 m, und/oder eine Breite oder einen Durchmesser von höchstens 0,2 m, vorzugsweise 0,1 m, aufweist.

3. Vorrichtung (2) nach einem der Ansprüche 1 oder 2, **dadurch gekennzeichnet, dass** die oder die wässrigen Lösungen der oder der Säulen jeweils eine saure Lösung, vorzugsweise mit einem pH-Wert von höchstens 5, und/oder eine basische Lösung, vorzugsweise mit einem pH-Wert von mindestens 9, umfassen.

4. Vorrichtung (2) nach einem der Ansprüche 1 bis 3, **dadurch gekennzeichnet, dass** die Einheit zur Geruchsneutralisation (20) mehrere Säulen mit wässriger Lösung (24, 26) umfasst. 26), wobei die Säulen in Reihe geschaltet sind, so dass die Gase, die in der wässrigen Lösung einer (24) der Säulen zirkuliert haben, oben aus der Säule (24) austreten und in den unteren Bereich einer anderen (26) der Säulen eingeführt werden, um in der wässrigen Lösung der Säule (26) zu zirkulieren; und/oder **dadurch gekennzeichnet, dass** die Gase in der oder den Säulen mit wässriger Lösung (24, 26) in Form von Blasen in aufsteigender Richtung unter dem Einfluss des Auftriebs zirkulieren; und/oder **dadurch gekennzeichnet, dass** die oder jede der Säulen mit wässriger Lösung (24, 26) Mittel umfasst, die es ermöglichen, ihre wässrige Lösung zu entleeren, insbesondere in den Abfallbehälter.

5. Vorrichtung (2) nach einem der Ansprüche 1 bis 4, **dadurch gekennzeichnet, dass** sie eine Plattform (4) umfasst, die den Behälter (6), die Entlüftungsmittel (16, 18, 114, 116, 118), den Einfülltrichter (8) und die Einheit zur Geruchsneutralisation (20) trägt, wobei die Plattform (4) so konfiguriert ist, dass sie auf einem LKW mit einem hydraulischen mechanischen Arm (32) geladen werden kann, wobei die Plattform (4) optional an einem Ende eine Öffnung (4³) umfasst, die geeignet ist, von einem Haken des mechanischen Arms des LKW (32) erfasst zu werden.

6. Vorrichtung (2) nach einem der Ansprüche 1 bis 5, **dadurch gekennzeichnet, dass** die Leitung (10, 10'), die den Einfülltrichter (8) mit dem Abfallbehälter (6) verbindet, in den Behälter (6) durch eine untere Wand (14') des Behälters mündet.

7. Vorrichtung (2) nach einem der Ansprüche 1 bis 5, **dadurch gekennzeichnet, dass** die Leitung (10, 10'), die den Einfülltrichter (8) mit dem Abfallbehälter (6) verbindet, wahlweise durch eine untere Wand (14') des Behälters oder durch eine obere Wand (14) des Behälters in den Behälter (6) mündet.

8. Vorrichtung (2) nach einem der Ansprüche 1 bis 7, **dadurch gekennzeichnet, dass** der Einfülltrichter (8) einen Abfallzerkleinerer (8²) umfasst; und/oder **dadurch gekennzeichnet, dass** die Vorrichtung (2) ein Hebe- und Kipp-System für Abfallbehälter umfasst, das geeignet ist, die Abfälle automatisch in den Einfülltrichter (8) zu entleeren.

9. Vorrichtung (2) nach einem der Ansprüche 1 bis 8, **dadurch gekennzeichnet, dass** sie eine elektrische Steuereinheit (22) umfasst, die so konfiguriert ist, dass sie die Vakuumpumpe (16) aktiviert, wenn der Unterdruck im Abfallbehälter (6) unter 0,5 bar, vorzugsweise unter 0,75 bar, liegt.

10. Vorrichtung (2) nach einem der Ansprüche 1 bis 9, **dadurch gekennzeichnet, dass** der Einfülltrichter (8) einen Deckel (8³) umfasst, der zwischen einer offenen und einer geschlossenen Position beweglich ist, und die Vorrichtung (2) ein Magnetventil (12) umfasst, das sich auf der Leitung (10, 10') befindet, die den Einfülltrichter (8) mit dem Abfallbehälter (6) verbindet, und elektrische Mittel (22) zur Steuerung des Magnetventils (12) umfasst.
mittel (22) sind so konfiguriert, dass sie die Öffnung des besagten Magnetventils (12) nur dann ermöglichen, wenn der Deckel (8³) in geschlossener Position ist.

11. Vorrichtung (2) nach einem der Ansprüche 1 bis 10, **dadurch gekennzeichnet, dass** der Schwellenwert zwischen 0,08 und 0,12 bar über dem atmosphärischen Druck liegt.

12. Vorrichtung (2) nach einem der Ansprüche 1 bis 11, **dadurch gekennzeichnet, dass** die Leitung (10) eine Schneckenwelle (112) oder eine peristaltische Pumpe umfasst und optional **dadurch gekennzeichnet, dass** der Einfülltrichter (8) einen Deckel (8³) umfasst, der zwischen einer offenen und einer geschlossenen Position beweglich ist, und die Vorrichtung elektrische Steuer- (22) und Antriebsmittel (110) für die besagte Schneckenwelle (112) oder die besagte peristaltische Pumpe umfasst, wobei die besagten Mittel (22, 110) so konfiguriert sind, dass sie die Schneckenwelle oder die peristaltische Pumpe nur dann in Rotation versetzen, wenn der Deckel (8³) in geschlossener Position ist.

13. Verfahren zur Lagerung von organischen Abfällen, wie Lebensmittelabfällen, umfassend die folgenden Schritte:
- Bereitstellung einer Vorrichtung zur Lagerung der Abfälle;
- Einfüllen der Abfälle in die Vorrichtung zur Lagerung;
**dadurch gekennzeichnet, dass** die Vorrichtung (2) gemäß einem der Ansprüche 1 bis 12 ist.

14. Verfahren nach Anspruch 13, **dadurch gekennzeichnet, dass** die Vorrichtung zur Lagerung (2) stationär gehalten wird, bis sie gefüllt ist, wobei die Standzeit es den Abfällen ermöglicht, sich zu zersetzen, wobei die Standzeit der Vorrichtung (2) optional mehr als 10 Tage, vorzugsweise 20 Tage, und noch vorzugsweise mehr als 30 Tage beträgt.

15. Verfahren nach einem der Ansprüche 13 bis 14, **dadurch gekennzeichnet, dass** es einen Schritt des Entfernens der Vorrichtung (2) umfasst, wenn sie einen vorbestimmten Füllstand erreicht, wobei das besagte Entfernen durch Beladen auf einen LKW mit einem hydraulischen mechanischen Arm (32) erfolgt.

## Claims

1. A device (2) for storage and/or transport of organic waste, such as food waste, the device comprising:
- a hermetic tank (6) intended to receive the waste;
- a discharge chute (8) for the waste, fluidically connected by a conduit (10, 10') to the waste tank;
- evacuation means (16, 18, 114, 116, 118) for the gases present in said tank,
further comprising,
an odor neutralization unit (20) for the gases emitted by said gases, the odor neutralization unit (20) being fluidically connected to the tank (6) by said evacuation means (16, 18, 114, 116, 118) and comprising at least one aqueous solution column (24, 26), the odor neutralization unit (20) being configured to receive the gases from the tank (6) at the bottom of said column or columns (24, 26),
the evacuation means for the gases comprising, in a first embodiment:
- a vacuum pump (16) fluidically connected (18) to the waste tank (6) and configured to suck the gases present in said tank and create a vacuum in said tank; or, in a second embodiment:
- an overpressure valve (114) configured to allow the gases from the tank (6) to pass to the odor neutralization unit (20) only when the gas pressure in the tank is greater than a threshold value;
the evacuation means for the gases further comprising, in the second embodiment, a check valve (116) allowing the circulation of gases only in the direction from the tank (6) to the odor neutralization unit (20).

2. The device (2) according to claim 1, **characterized in that** each aqueous solution column (24, 26) has a height greater than or equal to 0.5 m, preferably 1 m, and/or each aqueous solution column (24, 26) has a width or diameter less than or equal to 0.2 m, preferably 0.1 m.

3. The device (2) according to one of claims 1 or 2, **characterized in that** the aqueous solutions of the column or columns, respectively, comprise an acidic solution, preferably with a pH less than or equal to 5 and/or a basic solution, preferably with a pH greater than 9.

4. The device (2) according to one of claims 1 to 3, **characterized in that** the odor neutralization unit (20) comprises several aqueous solution columns (24, 26). 26), said columns being connected in series so that the gases having circulated in the aqueous solution of one (24) of the columns exit at the top of said column (24) and are introduced at the bottom of another (26) of said columns so as to circulate in the aqueous solution of said column (26); and/or **characterized in that** the gases circulate in the one or more aqueous solution columns (24, 26) in the form of bubbles following an upward direction under the effect of Archimedes' thrust; and/or **characterized in that** the one or each of the aqueous solution columns (24, 26) comprises means for draining its aqueous solution, notably into the waste tank.

5. The device (2) according to one of claims 1 to 4, **characterized in that** it comprises a platform (4) supporting the tank (6), the evacuation means for the gases (16, 18, 114, 116, 118), the discharge chute (8) and the odor neutralization unit (20), said platform (4) being configured to be loaded onto a skip truck (32) equipped with a hydraulic mechanical arm, the platform (4) optionally comprising, at one end, an orifice (4³) suitable for being engaged by a hook of the mechanical arm of the skip truck (32).

6. The device (2) according to one of claims 1 to 5, **characterized in that** the conduit (10, 10') connecting the discharge chute (8) to the waste tank (6) opens into said tank (6) through (14') a lower wall of said tank.

7. The device (2) according to one of claims 1 to 5, **characterized in that** the conduit (10, 10') connecting the discharge chute (8) to the waste tank (6) opens into said tank (6) selectively through (14') a lower wall of said tank or through (14) an upper wall of said tank.

8. The device (2) according to one of claims 1 to 7, **characterized in that** the discharge chute (8) comprises a waste grinder (8²); and/or **characterized in that** the device (2) comprises a system for lifting and tipping waste containers, suitable for automatically discharging said waste into the discharge chute (8).

9. The device (2) according to one of claims 1 to 8, **characterized in that** it comprises an electrical unit (22) for controlling the device configured to activate the vacuum pump (16) when the vacuum in the waste tank (6) is less than 0.5 bar, preferably 0.75 bar.

10. The device (2) according to one of claims 1 to 9, **characterized in that** the discharge chute (8) comprises a lid (8³) movable between an open position and a closed position, and the device (2) comprises a solenoid valve (12) located on the conduit (10, 10') connecting the discharge chute (8) to the waste tank (6), and electrical means (22) for controlling said solenoid valve (12), said
means (22) being configured to allow the opening of said solenoid valve (12) only when the lid (8³) is in the closed position.

11. The device (2) according to one of claims 1 to 10, **characterized in that** the threshold value is between 0.08 and 0.12 bar above atmospheric pressure.

12. The device (2) according to one of claims 1 to 11, **characterized in that** the conduit (10) comprises a screw conveyor (112) or a peristaltic pump and optionally **characterized in that** the discharge chute (8) comprises a lid (8³) movable between an open position and a closed position, and the device comprises electrical control means (22) and drive means (110) for said screw conveyor (112) or said peristaltic pump, said means (22, 110) being configured to drive the screw conveyor or the peristaltic pump in rotation only when the lid (8³) is in the closed position.

13. A method for storing organic waste, such as food waste, comprising the following steps:
- providing a waste storage device;
- discharging the waste into the storage device;
**characterized in that** the device (2) is in accordance with one of claims 1 to 12.

14. The method according to claim 13, **characterized in that** the storage device (2) is kept stationary until it is filled, the stationary period allowing the waste to decompose, the stationary period of the device (2) being optionally more than 10 days, preferably 20 days, more preferably more than 30 days.

15. The method according to one of claims 13 to 14, **characterized in that** it comprises a step of removing the device (2) when it reaches a predetermined fill level, said removal being carried out by loading onto a skip truck (32).
